(19) Europäisches Patentamt
European Patent Office
Office européen des brevets

(11) Veröffentlichungsnummer: **0 240 871**
**B1**

# (12) EUROPÄISCHE PATENTSCHRIFT

(45) Veröffentlichungstag der Patentschrift: **16.08.89**

(21) Anmeldenummer: **87104570.4**

(22) Anmeldetag: **27.03.87**

(51) Int. Cl.4: **C07C 41/18**, C07C 17/33, C07C 43/12, C07C 43/225, C07C 19/02

## (54) Verfahren zur Herstellung aliphatischer Chlorverbindungen.

(30) Priorität: **05.04.86 DE 3611419**

(43) Veröffentlichungstag der Anmeldung: **14.10.87 Patentblatt 87/42**

(45) Bekanntmachung des Hinweises auf die Patenterteilung: **16.08.89 Patentblatt 89/33**

(84) Benannte Vertragsstaaten: **BE CH DE FR GB IT LI NL**

(56) Entgegenhaltungen:
**EP-A- 0 025 829**
**EP-A- 0 180 356**
**EP-A- 0 200 403**
**DE-A- 2 545 659**
**US-A- 4 605 784**

(73) Patentinhaber: **BASF Aktiengesellschaft, Carl-Bosch-Strasse 38, D-6700 Ludwigshafen(DE)**

(72) Erfinder: **Greif, Norbert, Dr., Im Woogtal 3, D-6719 Bobenheim(DE)**
Erfinder: **Oppenlaender, Knut, Dr., Otto-Dill-Strasse 23, D-6700 Ludwigshafen(DE)**

**EP 0 240 871 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung aliphatischer Chlorverbindungen durch Decarboxylierung von Chlorkohlensäureestern oder -diestern bei erhöhten Temperaturen.

Die europäische Patentschrift 25 829 beschreibt die Herstellung von Alkylchloriden, wobei der Alkylrest auch durch Sauerstoffatome unterbrochen sein kann, durch Decarboxylierung von Chlorkohlensäureestern in Gegenwart von Trialkylamin-Hydrochloriden bei 90 - 170°C. Um gute Umsätze zu erzielen, müssen große Mengen des Amin-Hydrochlorids eingesetzt werden. Vorteilhaft wird die Decarboxylierung in einer Schmelze des Hydrochlorids durchgeführt und das Produkt destillativ aus dem rohen Reaktionsgemisch gewonnen. Bei der Herstellung nicht flüchtiger Alkylchloride soll die Katalysatorkonzentration nicht unter 3 Gew.-%, bezogen auf den Ausgangsstoff, abfallen. Setzt man Polyethergruppen enthaltende Chlorkohlensäureester ein und reduziert die Katalysatormenge auf 7,5 Gew.-%, bezogen auf den Ester, findet die Decarboxylierung zwar innerhalb von einigen Stunden statt, jedoch sind die entstehenden Chloride stark mit Nebenprodukten verunreinigt, wie die im Verlauf der Abbaureaktion aufgenommenen IR-Spektren zeigen. Die Verwendung großer Mengen an Ammoniumsalz hat zur Folge, daß im Falle nicht flüchtiger Produkte, bei denen eine direkte destillative Isolierung nicht möglich ist, eine technisch aufwendige wäßrige Extraktion zur Abtrennung der Salze erforderlich wird; außerdem treten Entsorgungsprobleme auf.

Der Erfindung lag daher die Aufgabe zugrunde, ein Verfahren zur Decarboxylierung von aliphatischen Chlorkohlensäureestern zu finden, bei dem geringe Katalysatormengen ausreichen, hohe Umsätze bei kurzen Reaktionszeiten möglich sind und die Bildung von Nebenprodukten weitgehend unterbleibt.

Demgemäß wurde ein Verfahren zur Herstellung von aliphatischen Chlorverbindungen der Formel

$$X\text{-}R^1\text{-}Cl \qquad I$$

in der $R^1$ eine Alkylenkette oder eine durch ein oder mehrere Sauerstoffatome unterbrochene Alkylenkette bedeutet, die durch Halogenatome, Alkyl- oder Arylgruppen substituiert sein kann, und X für Wasserstoff, Chlor oder den Rest $OR^2$ steht, wobei $R^2$ einen Alkyl-, Cycloalkyl-, Aryl- oder Aralkylrest darstellt, durch Decarboxylierung der entsprechenden Chlorkohlensäureester oder -diester bei erhöhter Temperatur, welches dadurch gekennzeichnet ist, daß man die Decarboxylierung in Gegenwart eines quartären Ammonium- oder Phosphoniumsalzes als Katalysator vornimmt.

Als Ausgangsstoffe kommen vorzugsweise Chlorkohlensäureester der Formel

$$R^4O\text{-}(CH_2\text{-}\underset{}{\overset{R^3}{\overset{|}{C}}}H\text{-}O)_k\text{-}(CH_2)_l\text{-}OCOCl \qquad II,$$

in der $R^4$ einen Alkyl-, Cycloalkyl-, Aryl- oder Aralkylrest oder den Rest -COCl oder $-(CH_2)_lOCOCl$ darstellt, $R^3$ für Wasserstoff, einen Phenylrest oder eine gegebenenfalls durch Halogen, z.B. durch Chlor oder Brom substituierte $C_1$- bis $C_4$-Alkylgruppe, z.B. die Methyl-, Ethyl-, Propyl-, Butyl- oder Chlormethylgruppe steht, k die Zahlen 0 bis 100, vorzugsweise 0 bis 50, insbesondere 0 bis 20 und l die Zahlen 2 bis 6 bedeuten, in Betracht.

Vorzugsweise steht $R^4$ für eine Alkylgruppe mit 1 bis 25, insbesondere 4 bis 20 Kohlenstoffatomen, eine $C_5$- oder $C_6$-Cycloalkylgruppe, eine Phenyl- oder Benzylgruppe oder eine alkylsubstituierte Phenylgruppe, z.B. eine Isopropyl-, mono-, di- oder tri-Butyl-, Amyl-, Octyl-, di-Nonyl- oder Dodecylphenylgruppe.

Ausgehend von Diestern, in denen $R^4$ den Rest -COCl oder $-(CH_2)_l\text{-}OCOCl$ bedeutet, werden die entsprechenden Dichloride erhalten.

Vorteilhaft lassen sich auch Chlorkohlensäureester der Formel

$$H\text{-}(CH_2)_m\text{-}OCOCl \qquad III,$$

in der m z.B. für die Zahlen 1 bis 30, insbesondere 1 bis 22 steht, oder Diester der Formel

$$ClOCO\text{-}(CH_2)_m\text{-}OCOCl \qquad IV$$

zu Alkylchloriden decarboxylieren. Die Alkylkette kann noch inerte Substituenten wie Halogenatome, Alkyl- oder Arylgruppen, insbesondere Chlor, Brom, $C_1$-$C_4$-Alkylgruppen oder Phenylgruppen tragen.

Die Ausgangsstoffe sind auf bekannte Weise, z.B. durch Umsetzung der entsprechenden Alkohole mit Phosgen erhältlich. Ihre Darstellung kann zweckmäßigerweise in situ erfolgen.

Die Decarboxylierung wird in Gegenwart eines quartären Phosphonium- oder Ammoniumsalzes durchgeführt. Ammoniumsalze sind z.B. solche der Formel

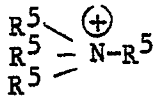 $Y^{\ominus}$ V,

in der die Reste $R^5$ gleiche oder verschiedene Kohlenwasserstoffreste mit je 1 bis 2O Kohlenstoffatomen bedeuten, z.B. $C_1$-$C_{20}$-Alkyl-, $C_5$- oder $C_6$-Cycloalkyl-, Aryl- wie die Phenyl- oder p-Tolylgruppe oder Alkylarylgruppen wie phenylsubstituierte $C_1$- bis $C_4$-Alkylgruppen und $Y^{\ominus}$ für die korrespondierende Base einer Mineralsäure, z.B. für $Cl^{\ominus}$, $Br^{\ominus}$, $J^{\ominus}$, $HSO_4^{\ominus}$, $NO_3^{\ominus}$, $HCO_3^{\ominus}$ oder $H_2PO_4^{\ominus}$ steht. Ferner können die Alkylreste $R^5$ auch miteinander verbunden sein, z.B. unter Ausbildung eines Piperidinringes. Besonders bevorzugt sind die für phasentransferkatalysierte Reaktionen üblichen quartären Ammonium- oder Phosphoniumsalze, z.B. Tetraalkyl- oder Trialkylbenzylammoniumsalze wie Triethyl- oder Tributylbenzylammoniumchlorid, Tetrabutylammoniumchlorid, -bromid oder -hydrogensulfat, Dimethyldicetylammoniumchlorid oder Methyltrioctylammoniumchlorid.

Die als Katalysator möglichen Phosphoniumsalze zeigen prinzipiell die gleiche Struktur wie V mit Phosphor anstelle von Stickstoff als positives Zentrum. Beispielsweise sei das Tributylhexadecylphosphoniumbromid genannt.

Zur Zersetzung der Chlorkohlensäureester können 0,05 bis 10 Gew.-% des Katalysators, bezogen auf den Chlorkohlensäureester, verwendet werden. Es sind bereits geringe Katalysatormengen von O,O3 bis 7, vorzugsweise O,15 bis zu 3, insbesondere O,1 bis O,5 Gew.-%, bezogen auf der Chlorkohlensäureester, ausreichend, so daß es möglich ist, die Endprodukte ohne weitere Aufarbeitung für Folgeumsetzungen zu verwenden.

Die Decarboxylierung kann bei Reaktionstemperaturen von 8O bis 17O, vorzugsweise 11O bis 14O, insbesondere 12O bis 13O°C ohne Lösungsmittel oder in Gegenwart eines inerten, aprotischen Lösungsmittels wie z.B. Methylenchlorid, Chloroform, Polyethern, Dioxan oder Toluol vorgenommen werden.

Die Durchführung des Verfahrens erfolgt zweckmäßigerweise derart, daß man den Ausgangsstoff, der ggf. in situ hergestellt wird, auf die Reaktionstemperatur erhitzt und den Katalysator hinzufügt. Der Reaktionsablauf kann z.B. IR-spektroskopisch verfolgt werden. In der Regel ist die Umsetzung nach 3O bis 12O min. beendet und die Reaktionsprodukte können in an sich bekannter Weise, z.B. destillativ im Falle flüchtiger Chloride oder im Falle nicht flüchtiger Chloride durch Extraktion und destilla tiver Reinigung isoliert werden. Bei Verwendung geringer Katalysatormengen ist eine Aufarbeitung häufig entbehrlich.

Die nach dem erfindungsgemäßen Verfahren erhaltenen Produkte können für Alkylierungsreaktionen z.B. für Sulfitalkylierungen zur Herstellung von Sulfonaten verwendet werden.

<u>Beispiel 1</u>

Herstellung von n-Chlordecan

Zu 1O3 ml n-Decanol wurden bei 1O°C 71 g einer 3O %igen Phosgen-Lösung in Toluol getropft und 1 h bei 3O°C nachgerührt. Anschließend wurde 1 g Methyltrioctylammonium-chlorid zugesetzt und das Reaktionsgemisch langsam auf 13O°C erwärmt, wobei $CO_2$ entwich und Toluol abdestilliert wurde. Der Rückstand wurde 2 h bei 13O°C gerührt. Im IR-Spektrum war die Carbonyladsorptionsbande bei 1777 $cm^{-1}$ praktisch verschwunden.

Analyse: Cl-Wert (gef.) = 19,2 % (= 96 % d. Th.)
Cl-Wert (theor.) = 2O,O8 %

<u>Beispiel 2</u>

Herstellung von 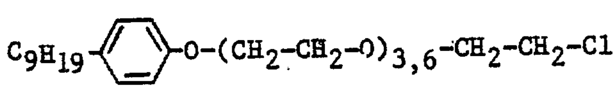

Zu 423 g

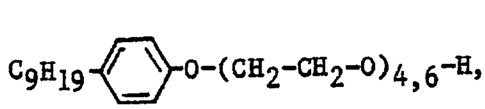

das 8O %ig in Methylenchlorid vorgelegt wurde, wurden 357 g einer 3O %igen Phosgen-Lösung in Toluol bei 1O°C zugetropft und 1 h bei 4O°C gerührt. Das Reaktionsgemisch wurde dann nach Zugabe von O,5 g (= O,1 % bez. auf das Ethoxilat) Methyltrioctylammonium-chlorid wie in Beispiel 1 beschrieben behandelt.

Analyse: Chlor (gef.) = 8,15 %
Chlor (theor.) = 8,O4 %

Beispiel 3

Herstellung von $Cl-CH_2-CH_2-(O-CH_2-CH_2)_8-O-CH_2-CH_2-Cl$

Zu 108 g Nonaethylenglycol wurden 55 g Phosgen in 150 ml Toluol bei 10°C zugetropft. Der gebildete Bischlorkohlensäureester wurde mit 0,21 g, Tributyl-hexadecylphosphoniumbromid versetzt und das Reaktionsgemisch wie in Beispiel 1 beschrieben behandelt.
Analyse: Cl-Wert (gef.) = 15 % (= 92 % d. Theorie)
Cl-Wert (theor.) = 16,24 %

**Patentansprüche**

1. Verfahren zur Herstellung von aliphatischen Chlorverbindungen der Formel

$X-R^1-Cl$ I

in der $R^1$ eine Alkylenkette oder eine durch ein oder mehrere Sauerstoffatome unterbrochene Alkylenkette bedeutet, die durch Halogenatome, Alkyl- oder Arylgruppen substituiert sein kann, und X für Wasserstoff, Chlor oder den Rest $OR^2$ steht, wobei $R^2$ einen Alkyl-, Cycloalkyl-, Aryl- oder Aralkylrest darstellt, durch Decarboxylierung der entsprechenden Chlorkohlensäureester oder -diester bei erhöhter Temperatur, dadurch gekennzeichnet, daß man die Decarboxylierung in Gegenwart eines quartären Ammonium- oder Phosphoniumsalzes als Katalysator vornimmt.

2. Verfahren nach Anspruch 1, dadurch gekennzeichnet, daß man Chlorkohlensäureester der Formel

$$R^4O-(CH_2-\overset{R^3}{\overset{|}{C}}H-O)_k-(CH_2)_l-OCOCl \qquad II,$$

in der $R^4$ den Rest $R^2$ oder den Rest -COCl oder $-(CH_2)_lOCOCl$ darstellt; $R^3$ für Wasserstoff, eine $C_1$-$C_4$-Alkylgruppe oder eine Phenylgruppe steht, k die Zahlen 0 bis 100 und l die Zahlen 2 bis 6 bedeuten, decarboxyliert.

3. Verfahren nach Anspruch 1, dadurch gekennzeichnet, daß man Chlorkohlensäureester der Formel

$H-(CH_2)_m-OCOCl$ III,

in der m eine Zahl von 1 bis 30 bedeutet, oder Diester der Formel

$ClOCO-(CH_2)_m-OCOCl$ IV

decarboxyliert.

4. Verfahren nach den Ansprüchen 1 bis 3, dadurch gekennzeichnet, daß man die Decarboxylierung in Gegenwart eines Ammoniumsalzes der Formel

$$\left[(R^5)_3\overset{\oplus}{N}-R^5\right] \quad Y^{\ominus} \qquad V,$$

in der die Reste $R^5$ gleiche oder verschiedene Kohlenwasserstoffreste mit je 1 - 20 Kohlenstoffatomen bedeuten, und Y die korrespondierende Base einer Mineralsäure darstellt, vornimmt.

5. Verfahren nach Anspruch 4, dadurch gekennzeichnet, daß man ein Tetraalkyl- oder Benzyltrialkylammoniumsalz verwendet.

6. Verfahren nach den Ansprüchen 1 bis 3, dadurch gekennzeichnet, daß man die Decarboxylierung in Gegenwart von Tributylhexadecylphosphoniumbromid vornimmt.

7. Verfahren nach den Ansprüchen 1 bis 6, dadurch gekennzeichnet, daß man 0,05 bis 10 Gew.-% des Katalysators, bezogen auf den Chlorkohlensäureester, verwendet.

8. Verfahren nach den Ansprüchen 1 bis 7, dadurch gekennzeichnet, daß man die Decarboxylierung bei Temperaturen von 80 bis 170°C vornimmt.

## Claims

1. A process for preparing an aliphatic chlorine compound of the formula

$X-R^1-Cl$, I

where $R^1$ is alkylene which may be interrupted by one or more oxygen atoms and which may be substituted by halogen, alkyl or aryl, and X is hydrogen, chlorine or $OR^2$, where $R^2$ is alkyl, cycloalkyl, aryl or aralkyl, by decarboxylating the corresponding chloroformic ester or diester at elevated temperatures, which comprises decarboxylating in the presence of a quaternary ammonium or phosphonium salt as catalyst.

2. A process as claimed in claim 1, wherein a chloroformic ester of the formula

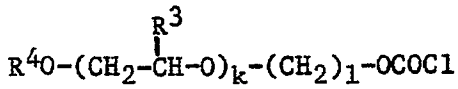 II,

where $R^4$ is $R^2$, $-COCl$ or $-(CH_2)_lOCOCl$, $R^3$ is hydrogen, $C_1$–$C_4$-alkyl or phenyl, k is 0 to 100 and l is 2 to 6, is decarboxylated.

3. A process as claimed in claim 1, wherein a chloroformic ester of the formula

$H-(CH_2)_m-OCOCl$, III

where m is 1 to 30, or a diester of the formula

$ClOCO-(CH_2)_m-OCOCl$, IV

is decarboxylated.

4. A process as claimed in any of claims 1 to 3, wherein the decarboxylation is carried out in the presence of an ammonium salt of the formula

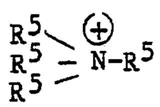 $Y^{\ominus}$ V,

where the radicals $R^5$ are identical or different hydrocarbon radicals of 1–20 carbon atoms each and Y is the corresponding base of a mineral acid.

5. A process as claimed in claim 4, wherein a tetraalkylammonium or benzyltrialkylammonium salt is used.

6. A process as claimed in any of claims 1 to 3, wherein the decarboxylation is carried out in the presence of tributylhexadecylphosphonium bromide.

7. A process as claimed in any of claims 1 to 6, wherein from 0.05 to 10% by weight of the catalyst, based on the chloroformic acid ester, is used.

8. A process as claimed in any of claims 1 to 7, wherein the decarboxylation is carried out at from 80 to 170°C.

## Revendications

1. Procédé de préparation de composés chlorés aliphatiques de formule

$X-R^1-Cl$, I

dans laquelle $R^1$ est une chaîne alkylène ou une chaîne alkylène interrompue par un ou plusieurs atomes d'oxygène qui peut être substituée par des atomes d'halogène ou des groupements alkyle ou aryle, et X est mis pour un atome d'hydrogène ou de chlore ou pour le reste $OR^2$, où $R^2$ représente un reste alkyle, cycloalkyle, aryle ou aralkyle, par décarboxylation de l'ester ou du diester d'acide carboxylique chloré correspondant à température élevée, caractérisé en ce qu'on mène la décarboxylation en présence d'un sel d'ammonium ou de phosphonium quaternaire comme catalyseur.

2. Procédé selon la revendication 1, caractérisé en ce qu'on décarboxyle un ester d'acide carboxylique chloré de formule

$$R^4O-(CH_2-\underset{|}{\overset{R^3}{C}}H-O)_k-(CH_2)_l-OCOCl \qquad \text{II},$$

dans laquelle $R^4$ représente le reste $R^2$ ou le reste $-COCl$ ou $-(CH_2)_lOCOCl$; $R^3$ est mis pour un atome d'hydrogène, un groupement alkyle en $C_1-C_4$ ou un groupement phényle, k représente les nombres de 0 à 100 et l représente les nombres de 2 à 6.

3. Procédé selon la revendication 1, caractérisé en ce qu'on décarboxyle un ester d'acide carboxylique chloré de formule

$H-(CH_2)_m-OCOCl$, III

dans laquelle m représente les nombres de 1 à 30, ou un diester de formule

$ClOCO-(CH_2)_m-OCOCl$, IV

4. Procédé selon les revendications 1 à 3, caractérisé en ce qu'on mène la décarboxylation en présence d'un sel d'ammonium de formule

$$\begin{matrix} R^5 \diagdown \\ R^5 - \\ R^5 \diagup \end{matrix} \overset{\oplus}{N}-R^5 \qquad Y^{\ominus} \qquad \text{V},$$

dans laquelle le reste $R^5$ représente des restes hydrocarbonés identiques ou différents avec chacun de 1 à 20 atomes de carbone, et Y représente la base correspondante d'un acide minéral.

5. Procédé selon la revendication 4, caractérisé en ce qu'on utilise un sel de tétraalkyl- ou de benzyltrialkylammonium.

6. Procédé selon les revendications 1 à 3, caractérisé en ce qu'on mène la décarboxylation en présence de bromure de tributylhexadécylphosphonium.

7. Procédé selon les revendications 1 à 6, caractérisé en ce qu'on utilise de 0,05 à 10% en poids du catalyseur, par rapport à l'ester d'acide carboxylique chloré.

8. Procédé selon les revendications 1 à 7, caractérisé en ce qu'on mène la décarboxylation à des températures de 80 à 170°C.